# DEMANDE DE BREVET EUROPEEN

(11) **EP 2 491 950 A1**
(43) Date de publication de la demande: **29.08.2012**
(21) Numéro de dépôt: 11360010.0
(22) Date de dépôt: 25.02.2011
(51) Int. Cl.: A61K 41/00, A61K 9/16, C12N 15/11

(54) **Préparations homéopathiques à base d'ARNi.**

(71) Demandeur: Con.Form-Bimureg, 68230 Walbach (FR)
(72) Inventeur: M. Glady, Gilbert, 68230 Walbach (FR)
(74) Mandataire: Barny, Luc

(57) **Abrégé**

La présente invention porte sur l'utilisation thérapeutique d'acides ribonucléiques interférents. Cette invention décrit un procédé de fabrication de préparations pharmaceutiques selon la méthodologie homéopathique, contenant un ou plusieurs acides ribonucléiques interférents. Sont également décrites des préparations pharmaceutiques obtenues par la mise en oeuvre dudit procédé ainsi que l'utilisation de telles préparations homéopathiques pour le traitement et ou la prévention de pathologies humaines ou animales.

## Description

La présente invention porte sur l'utilisation thérapeutique d'acides ribonucléiques interférents. Cette invention décrit un procédé de fabrication de préparations pharmaceutiques selon la méthodologie homéopathique, contenant un ou plusieurs acides ribonucléiques interférents. Sont également décrites des préparations pharmaceutiques obtenues par la mise en oeuvre dudit procédé ainsi que l'utilisation de telles préparations homéopathiques pour le traitement et ou la prévention de pathologies humaines ou animales.

Les acides ribonucléiques (ci-après « ARN » en français ou « RNA » en anglais) sont des polymères constitués de nucléotides. Les nucléotides sont composés d'une base hétérocyclique azotée, d'un pentose en l'occurrence le ß-D-ribose, et d'un acide phosphorique. On trouve les ARN principalement dans le cytoplasme des cellules. Par ailleurs, on distingue différents types d'ARN qui interviennent dans l'expression de l'information génétique. On citera notamment l'ARN ribosomique (ARNr), l'ARN de transfert (ARNt), l'ARN messager (ARNm) ainsi que les ARN non codants. Récemment, on a mis en évidence un nouveau type d'ARN appelée ARN interférent. Les acides ribonucléiques interférents, également appelés ARNi, sont de petits acides nucléiques qui se lient spécifiquement avec l'ARN messager cible. Cette liaison spécifique conduit à la dégradation totale ou partielle de l'ARN messager cible et ainsi à l'inhibition de l'expression de la protéine correspondante. En effet, les ARN messagers sont les produits de transcription de l'ADN et ils transportent l'information génétique vers une zone de traduction où la protéine correspondante est synthétisée. Intervenir durant cette étape de transmission de l'information génétique de façon ciblée permet d'envisager d'empêcher la synthèse d'une protéine particulière.

Il est communément accepté que le traitement de pathologies humaines soit entrepris par l'administration de doses plus ou moins importantes de composés sélectionnés pour leurs effets opposés ou inhibiteurs des causes et/ou des symptômes de ladite pathologie. Cependant, ce principe présente parfois un manque d'efficacité et trop souvent des effets secondaires tels de simples gênes jusqu'à des intolérances voire des réactions allergiques. Dans certaines pathologies lourdes, telles le syndrome immunodéficitaire acquis (SIDA ou AIDS) ou certaines formes de cancer, il est même communément accepté que des médicaments soient administrés pour diminuer les effets secondaires liés à la prise des médicaments principaux visant à traiter la pathologie principale. Ce sont les doses de ces composés qui sont déterminantes pour l'efficacité du traitement.

Il est donc souhaitable de pouvoir proposer d'autres alternatives thérapeutiques qui soient basées sur de nouveaux principes. L'approche homéopathique est l'une de ces approches qui vise à stimuler l'organisme de l'hôte, humain ou animal, suite à une administration d'un composé thérapeutique hautement dilué. Ce principe permet notamment d'éviter l'apparition d'effets secondaires et d'assurer un meilleur confort au patient.

Le brevet européen EP0655928 (ETIENNE Marie-Christine) décrit des compositions homéopathiques ainsi que leur utilisation pour la préparation de médicaments destinés au traitement de maladies causées par des erreurs métaboliques et de certaines affections virales. Ce brevet décrit en particulier un produit homéopathique de formule générale RxCH où R désigne un principe actif et où CH désigne sa dilution homéopathique. Ce produit est utilisé en tant que médicament destiné à provoquer l'élimination hors des cellules qui le contiennent d'un composé de nature identique à celle dudit principe actif dans des cas où cette élimination a pour effet de rétablir le fonctionnement normal des systèmes transporteurs péricellulaires perturbés. Sont notamment cités comme groupe R : NaCl, fer réduit, sulfure noir, or, potassium, aluminium, etc. On prépare donc une solution homéopathique contenant le composé à éliminer des cellules atteintes par la pathologie que l'on envisage de traiter ou de prévenir. Le principe du traitement homéopathique de certaines pathologies est exposé dans ce brevet, cependant il ne considère pas une intervention au niveau du patrimoine génétique de l'organisme ainsi traité. Le brevet européen EP0670164 (LABO' LIFE®) décrit un procédé de préparation de solutions homéopathiques contenant un acide nucléique et destinées à traiter ou à prévenir une pathologie infectieuse ou une pathologie impliquant le dysfonctionnement d'un gène. Les acides nucléiques sont de simples brins de longueur au moins égale à 10 nucléotides et dont la séquence est sensiblement analogue à celle d'un polynucléotide choisi parmi une partie du matériel génomique de l'agent infectieux en cas de traitement d'une maladie infectieuse ou une partie de la séquence liée au dysfonctionnement dans l'autre cas, le produit de transcription de ladite partie ou son produit de transcription inverse. Les acides nucléiques selon l'invention sont donc choisis de façon aléatoire en fonction de la pathologie à traiter :
- pour traiter une pathologie infectieuse on sélectionne dans l'ensemble du matériel génomique de l'agent infectieux un acide nucléique simple brin dont la longueur est au moins égale à 10 nucléotides ;
- pour traiter une pathologie liée au dysfonctionnement d'un gène, on sélectionne dans une partie de la séquence nucléotidique liée au dysfonctionnement, un acide nucléique simple brin dont la longueur est au moins égale à 10 nucléotides.

Les produits de transcription et/ou de transcription inverse sont également concernés. Les solutions homéopathiques sont préparées selon les méthodes connues de l'homme de l'art. Les acides nucléiques simples brins contenus dans les solutions homéopathiques selon l'invention ont pour action de provoquer l'élimination par les cellules traitées des produits issus du matériel génétique dont lesdits acides nucléiques ont une séquence sensiblement analogue. Le principe du traitement est basé sur le fait que les cellules ainsi traitées vont avoir une mémoire de ce qui est anormal ou néfaste pour elles suite à l'exposition aux acides nucléiques sélectionnés, engendrée par la solution homéopathique. C'est le même principe de « chasse » que pour le brevet EP0655928 (ETIENNE Marie-Christine). L'un des inconvénients majeurs de cette méthode est le choix de l'acide nucléique servant de « principe actif ». En effet, ce choix n'est pas spécifique et laisse une grande part au hasard. Dans le cas d'une pathologie liée au dysfonctionnement d'un gène, choisir un acide nucléique simple brin dont la longueur est au moins égale à 10 nucléotides revient à faire une sélection au hasard. Il est peu probable que le principe de la méthodologie homéopathique fonctionne avec cette importante part laissée au hasard.

Le phénomène d'interférence ARN a été mis en évidence par Jorgensen et al. (Napoli, C., Lemieux, C., Jorgensen, R. Plant Cell, 1990, 2(4) : 279-289) en 1990 lors d'une étude visant à déterminer le rôle exact de la chalcone synthase (CHS), une enzyme clé dans la biosynthèse des flavonoïdes, dans la synthèse des anthocyanes de pétunias. Les anthocyanes sont responsables de la coloration pourpre de ces fleurs. L'objectif était de renforcer la couleur pourpre des pétunias en surexprimant la chalcone synthase (CHS) à l'aide d'un vecteur, favorisant ainsi une surexpression du pigment responsable de la couleur pourpre. Suite à cette manipulation de leur patrimoine génétique, certaines plantes présentent des fleurs qui sont partiellement ou totalement blanches : 42% des fleurs dans lesquelles le gène CHS a été surexprimé sont blanches ou anormalement pâles contre 9% des fleurs contrôle dont le patrimoine génétique n'est pas modifié. L'analyse des ARNs isolés à partir des fleurs blanches ainsi obtenues a montré que le taux d'ARN messagers (ARNm) produit est fortement réduit par rapport aux taux d'ARN des fleurs contrôle. Par ailleurs, après analyse du patrimoine génétique de toutes les fleurs ainsi que de la séquence introduite dans le vecteur, il s'avère que les séquences d'ADN ne sont pas altérées. Ceci démontre clairement un dysfonctionnement de la biosynthèse des flavonoïdes alors que l'enzyme clé dans la synthèse des pigments de couleur aurait dû être surexprimée. Il semble donc que le matériel génétique introduit par le vecteur ait littéralement éteint, ou en tous les cas atténué, l'expression du gène naturel sans pour autant altérer ce gène naturel. On nommera ce phénomène la « co-suppression ». Par la suite, on parlera de gène rendu silencieux ou encore de phénomène de "transcriptional gene silencing" ou TGS.

Ce mécanisme sera ensuite observé dans d'autres règnes et notamment chez *Caenorhabditis elegans* (Fire A., Xu S., Montgomery M.K., Kostas S.A., Driver S.E., Mello, Nature, 1998, 391(6669):744-746) puis chez la drosophile (Kennerdell J.R., Carthew R.W., Nat. Biotechnol. 2000, 18(8):896-8 et Cogoni C, Macino G, Curr. Opin. Genet. Dev. 2000, 10 (6) : 638-43)*,* et les champignons (Catalanotto C., Azzalin G., Macino G., Cogoni C., Nature, 2000, 16, 404(6775):245). Fire A. et Mello C. ont obtenu le prix Nobel 2006 de physiologie et de médecine pour leurs travaux sur l'ARN interférence.

Le phénomène d'ARN interférence représente un formidable outil pour envisager d'éteindre l'expression d'un gène de façon ciblée. Il peut également être envisagé de simplement diminuer l'expression d'un gène de façon ciblée sans pour autant l'éteindre. En conséquence, il semble possible d'utiliser les ARN interférents pour inhiber de façon ciblée l'expression de matériel génétique. Ceci présente un intérêt particulier pour l'étude de la fonction des gènes mais ceci peut également être très intéressant pour le traitement et/ou la prévention de nombreuses pathologies dans lesquelles la neutralisation ciblée, totale ou partielle, de l'expression de matériel génétique pourrait avoir un intérêt. On citera en particulier les pathologies caractérisées par une surexpression de protéines ou l'apparition de mutations aberrantes telles le cancer, les maladies neurodégénératives, certaines pathologies oculaires et certaines infections virales, etc..

La présente invention propose un procédé de fabrication de préparations pharmaceutiques selon la méthodologie homéopathique, contenant un ou plusieurs acides ribonucléiques interférents. Sont également décrites des préparations pharmaceutiques obtenues par la mise en oeuvre dudit procédé ainsi que l'utilisation de telles préparations homéopathiques pour le traitement et ou la prévention de pathologies humaines ou animales.

Les ARNs interférents peuvent être de plusieurs natures. En effet, parmi les ARN interférents on trouve les microARNs également appelés ARNmi ou miRNAs (microRNAs), mais également les petits ARN inteférents également appelés ARNsi ou siRNAs (pour « small interfering RNAs ») et enfin les ARNs courts en épingle à cheveux également appelés ARNsh ou shRNAs (pour « small hairpin RNAs »). Les ARNmi et les ARNsi sont de petits nucléotides d'une vingtaine de bases qui ne sont pas codés par la même partie du génome. Il a été constaté, notamment chez C. *elegans*, que suite à l'introduction d'ARN double brin dans une cellule, une enzyme appelée DICER va cliver cet ARN en petits fragments d'une vingtaine de résidus. Ceci semble être un mécanisme ancestral de défense contre les virus à ARN. C'est ce même mécanisme qui est aujourd'hui appelé ARN interférence.

En effet, les ARNmi sont les résultats de l'expression de gènes qui leur sont propres et dont ils sont les uniques produits. Leur transcrit est en premier lieu une molécule d'ARN simple brin d'une taille équivalent à un millier de nucléotides et chacun présente le long de sa séquence des motifs nucléotidiques intrachaînes partiellement complémentaires qui engendrent une structure secondaire en forme d'épingle à cheveux, on parlera alors d'ARN en épingle à cheveux ou ARNsh (pour « short hairpin RNA »). Les chercheurs définissent trois parties dans cette forme en épingle à cheveux : le « corps » qui est formé de séquences plus ou moins complémentaires présentes sur le simple brin et qui se disposent en vis-à-vis par phénomène d'appariement de bases, la « tête » en forme de boucle où il n'y a pas d'appariement et les « jambes » qui sont constituées par de simples brins et portent les séquences qui n'ont pas trouvé de bases complémentaires pour s'apparier. S'ensuit l'entrée en action des enzymes Drosha et Dicer qui sont des ribonucléases de type III et de l'exportin-5. L'enzyme Drosha effectue une première série de coupures qui réduit la molécule de shARN en l'amputant de ses « jambes » en une courte molécule d'une centaine de nucléotides. La protéine Exportin-5 va alors transporter ce shARN dans le cytoplasme. C'est à cet endroit que l'enzyme Dicer va effectuer une deuxième série de coupures qui va amputer le shARN de sa « tête » par réduction des séquences non appariées. Dans le même temps, des protéines de type hélicases associées au Dicer, vont dissocier l'ARN double brin subsistant après clivage des « jambes » et de la « tête » en deux molécules d'ARN simple brin. L'un des simples brins obtenus est un ARNmi.

Les ARNsi peuvent provenir d'une source endogène ou exogène. La source endogène est la transcription des parties exoniques d'un gène d'une taille plus étendue et la source exogène est soit un apport extérieur d'un ARN double brin par injection, soit un apport en provenance de génomes à ARN double brin (virus à ARN de plantes par exemple). L'enzyme Dicer clive et sépare les longs ARN double brin des ARNsi en fragments d'environ 20 nucléotides.

Les ARN simples brins obtenus après action du DICER sont des microARN (ARNmi) ou des petits ARN interférents (ARNsi). Ces ARNi s'associent à un complexe protéique appelé RISC (pour « RNA-induced silencing complex ») et c'est alors qu'il va agir sur l'ARNm cible auquel il s'apparie. En cas d'appariement parfait, généralement avec un ARNsi, l'ARNm est détruit et il n'y a pas de traduction. Par ailleurs, les protéines du complexe RISC qui participent activement à cette destruction restent parfaitement fonctionnelles. Ceci leur permet d'opérer à nouveau sur d'autres ARNm cible de même spécificité. Dans certains cas, on peut choisir un siRNA capable de cliver un ARNm porteur d'une mutation ponctuelle sans affecter l'ARNm sauvage. En cas d'appariement imparfait, généralement avec un ARNmi, les interactions se produisent via une protéine nommée Argonaute, qui est une enzyme appartenant au complexe protéique RISC. Il s'ensuit que le complexe protéique RISC, associé à un fragment de miARN, va catalyser un ou plusieurs clivage(s) au sein de la séquence complémentaire de l'ARNm cible. Ainsi, l'ARNm modifié sera détruit, supprimant ainsi l'expression de la protéine correspondante.

Les travaux de l'équipe de Robert Darnell à l'Université Rockefeller de New York, ont permis d'établir un catalogue de l'ensemble des sites d'interactions des microARN avec les ARN messagers exprimés dans un organe, en l'occurrence le tissu cérébral de la souris. Il s'est appuyé sur une méthode qu'il a mise au point récemment : la technique de séquençage à haut débit d'ARNs isolés par immunoprécipitation après pontage covalent, également appelée HITS-CLIP (pour « *High-Throughput Sequencing of RNAs isolated by CrossLinking ImmunoPrecipitation* »). La mise en oeuvre de cette technique leur a premis d'identifier plus de 450 miARNs à ce jour. Une analyse bioinformatique a ensuite mis en évidence les sites d'interactions entre un type de miARN et ses cibles. Ces informations ont permis d'établir un catalogue des vingt miARN les plus abondants dans le tissu cérébral de souris. L'analyse de ces vingt miARN a montré que les ARNm cibles de ces miARN correspondent à des gènes impliqués dans la différenciation des neurones et la régulation du fonctionnement du squelette cellulaire (Chi et al., Nature, 2009, 460 (7254) : 479-486)*.* Il peut donc maintenant être envisagé de reproduire ce type d'études pour tous les tissus et l'étendre à d'autres espèces.

La présente invention pallie les problèmes évoqués ci-dessus en combinant le potentiel d'action des ARN interférents et les potentialités d'une approche homéopathique afin de traiter et/ou prévenir certaines pathologies.

La présente invention porte sur une composition pharmaceutique préparée selon la méthodologie homéopathique contenant un ou plusieurs acides ribonucléiques interférents (ARNi). Ces ARNi sont des molécules simple brin ou double brin qui ont une taille comprise entre 20 et 22 nucléotides. Dans un mode de réalisation spécifique, la composition pharmaceutique selon l'invention contient un ARN interférent (ARNi) double brin. Dans un autre mode de réalisation, la composition pharmaceutique selon l'invention contient un ARN interférent (ARNi) simple brin. Enfin, dans un mode de réalisation préféré, la composition pharmaceutique selon l'invention contient un ARNi dont la taille est comprise entre 20 et 22 nucléotides, c'est-à-dire que la taille de cet ARNi peut être de 20, 21 ou 22 nucléotides.

Par **ARN interférents** (ARNi ou RNAi) on entend les miARNs, les siARNs et les shARNs ainsi que les gènes codants ces ARN interférents ; les pri-miARNs ; les pre-miARNs ; les ribonucléases de type III DROSHA et PASHA/DGCR8 ; les protéines EXPORTIN-5 et RAN GTP ; la ribonucléase de type III DICER et RLC ; le complexe protéique RISC (pour « RNA-induced silencing complex ») ; les protéines Argonautes RDE-1, QDE2, AGO1, AGO2, AGO3 et AGO4 les protéines VIG, fragile X-related protein et nuclease Tudor-SN ; les protéines elF2C2, helicase GEMIN3 et GEMIN4 ; les duplexes miARN:miARN, ainsi que les duplexes siARN : siARN.

Par **composition pharmaceutique** on entend une composition comprenant une substance active, en l'occurrence un ou plusieurs ARN interférents selon l'invention, et un ou plusieurs excipients pharmaceutiquement acceptables.

Par **méthodologie homéopathique** on entend la mise en oeuvre des principes décrits par Hahnemann ou par la suite Korsakov. Ces méthodes sont basées sur le principe d'administration aux patients de doses faibles voire infinitésimales d'un principe actif, lesdites doses étant obtenues par dilution et agitation, encore appelée dynamisation. On retrouvera ces mêmes principes dans G. NETIEN et al.: "Galenica Médicaments Homéopathiques. Notions pratiques de pharmacie homéopathique", édition 2, 1986. La méthodologie homéopathique adapte le traitement à l'état physique et psychique de chaque patient. Par **patient** on entend un humain ou un animal atteint d'une pathologie susceptible d'être traitée ou prévenue par une composition selon l'invention.

Par **succussion** on entend en homéopathie une méthode pour dynamiser les substances. Ce procédé a été décrit par Hahneman. Les substances à dynamiser sont en solution dans de l'eau, de l'alcool, un mélange des deux précédents ou encore dans du lactose. En général, l'alcool est le plus répandu des solvants mais suivant la nature de la substance à dynamiser, l'eau peut être le seul solvant utilisable. Durant les dilutions, le préparateur procède à l'agitation des solutions pour en mélanger les composants. Ces agitations ne peuvent se faire qu'à la main selon les prescriptions d'Hahneman. La succussion résulte d'une agitation violente, à la main, de la solution à chaque étape de dilution.

Les maladies concernées par l'invention sont à titre indicatif et non limitatif: les maladies génétiques, les maladies métaboliques, les maladies auto-immunes, les maladies virales, les maladies néoplasiques et les intoxications. De manière plus générale, les compositions pharmaceutiques selon l'invention permettent de traiter et/ou de prévenir les maladies infectieuses et les pathologies impliquant le dysfonctionnement d'un gène.

La présente invention décrit l'utilisation en thérapeutique humaine et vétérinaire de solutions contenant des molécules d'ARN interférent, préparées selon la méthodologie homéopathique de dilution-succussion tel que décrite à l'heure actuelle dans la réglementation régissant la pharmacopée européenne. Comme détaillé ci-dessus les ARNi sont impliqués de façon naturelle dans des processus biologiques ayant lieu à l'intérieur des cellules avec un effet soit d'activation, soit d'inhibition de l'expression protéique de certains gènes. Tout dérèglement à ce niveau va engendrer un processus biologique qui va éventuellement résulter en un état pathologique. C'est dans ce cas que les ARNi vont avoir un intérêt tout particulier. En effet, les ARNi en dilutions homéopathiques vont être capables de moduler l'action d'ARNm pour piloter l'activité transcriptionnelle de gènes impliqués dans une pathologie donnée. Cette action modulatrice est décrite par la loi d'Arndt-Schultz ou loi d'inversion d'action selon la dilution qui stipule que toute substance, au fil des dilutions qu'elle subit, acquiert des propriétés inverses de celles qui étaient les siennes dans son état initial. Ceci signifie donc qu'une substance ayant un effet X à l'état naturel est susceptible d'acquérir un effet 1/X à compter d'une certaine dilution. Il est à noter que des effets intermédiaires 1/Xⁿ seront observables dans des dilutions antérieures. En fonction de cette loi d'Arndt-Schultz, l'effet des ARNi peut être modulé quasiment à l'infini selon les objectifs thérapeutiques à atteindre. Les actions physiologiques de certains ARNi étant connues, chaque objectif thérapeutique sera défini en fonction de cette action physiologique et du patient à traiter.

Comme décrit ci-dessus, de nombreux ARNmi ont été identifiés et leur action physiologique a été clairement déterminée. Même s'il reste beaucoup de travail pour répertorier et identifier tous leurs rôles, les ARNi vont permettre de traiter de nombreuses pathologies avec une grande efficacité puisque leur action sera ciblée et leur quantité pourra être contrôlée. Les ARNi sont des molécules connues, identifiées et validées dont l'action est très spécifique. Le message qui leur est associé suite au processus de dilution-succussion va par conséquent être aisément identifié par la/les cellule(s) à traiter et les ARNm cibles qu'elle(s) contien(en)t. Pour toutes ces raisons on peut caractériser la présente invention comme un procédé de régulation immunogénétique biomimétique.

Dans un mode de réalisation particulier, l'invention porte sur une composition pharmaceutique selon l'invention contenant un ARNi choisi parmi les microARN (ARNmi), les petits ARN interférents (ARNsi) et les ARN en épingle à cheveux (ARNsh). Comme décrit précédemment un ARNsh a une taille bien supérieure à l'intervalle défini de 20 à 22 nucléotides pour les ARNmi et les ARNsi.

Par conséquent, dans un mode de réalisation préféré, la composition pharmaceutique selon l'invention contient un ARNi choisi parmi les microARN (ARNmi) et les petits ARN interférents (ARNsi).

Suivant le patient et la pathologie à traiter, on pourra envisager d'utiliser plutôt un microARN (ARNmi) ou plutôt un petit ARN interférent (ARNsi). En conséquence, dans un mode de réalisation particulier, la composition pharmaceutique selon l'invention contient un ou plusieurs microARNs (ARNmi). Dans un autre mode de réalisation particulier, la composition pharmaceutique selon l'invention contient un ou plusieurs petit(s) ARN(s) de transfert (ARNsi).

Dans un mode de réalisation particulier les compositions pharmaceutiques selon l'invention sont appelées BIMUREG® et les microARN (ARNmi) qui les composent sont sélectionnés parmi les suivants :

| BIMUREG® | microARN (ARNmi) entrant dans la composition |
|---|---|
| AAN 1+2 | miR-92 : microRNA-92 |
| | miR-147 : microRNA 147 |
| | miR-1 : microRNA-1 |
| | miR-135a : microRNA-135a |
| AAS Al+2+3 | miR-let-7 : microRNA-let-7 |
| AAS Cl+2+3 | miR-21 : microRNA-21 |
| | miR-26a : microRNA-26a |
| AAT 1+2+3 | miR-21 : microRNA-21 |
| | miR-126 : microRNA 126 |
| | miR-145 : microRNA 145 |
| | miR-155 : microRNA-155 |
| AAZ 1+2+3 | miR-29a/miR-29b-1 : microRNA-29a/microRNA-29b-1 |
| | miR-34 : microRNA-34a |
| | miR-107 : microRNA-107 |
| | miR-125b : microRNA-125b |
| | miR-328 : microRNA-328 |
| ACI 1+2 | miR-21 : microRNA-21 |
| | miR-375 : microRNA-375 |
| | miR-192 : microRNA-192 |
| AHT 1+2+3 | miR-155 : microRNA-155 |
| AIF 1+2+3 | miR-146a : microRNA-146a |
| AIFec 1+2+3 | miR-132 : microRNA-132 |
| | miR-146 : microRNA-146 |
| ANADI 1+2+3 | miR-30a-5p : microRNA-30a-5p |
| | miR-195 : microRNA-195 |
| | miR-103 : microRNA-103 |
| | miR-143 : microRNA-143 |
| | miR-519d : microRNA-519d |
| ANANEM 1+2+3 | miR-let-7d : microRNA-let-7d |
| APOPMIR-PLUS | miR-let7/miR-98 : microRNA-let7/microRNA-98 |
| | miR-1 : microRNA-1 |
| | miR-29a,b,c : microRNA-29a,b,c |
| | miR-34a,b,c : microRNA-34a,b,c |
| | miR-15/16 : microRNA-15/16 |
| | miR-101 : microRNA-101 |
| | miR-215 : microRNA-215 |
| | miR-192 : microRNA-192 |
| | miR-127 : microRNA-127 |
| APOPMIR-MINUS | miR-17-92 : microRNA-17-92 |
| | miR-21 : microRNA-21 |
| | miR-106b-25 : microRNA-106b-25 |
| | miR-133 : microRNA-133 |
| | miR-143 : microRNA-143 |
| | miR-145 : microRNA-145 |
| | miR-155 : microRNA-155 |
| | miR-206 : microRNA-206 |
| | miR-210 : microRNA-210 |
| | miR-221/222 : microRNA-221/222 |
| | miR-330 : microRNA-330 |
| APREGAN 1+2 | miR-30a-5p : microRNA-30a-5p |
| | miR-195 : microRNA-195 |
| APREGOR 1+2 | miR-30a-5p : microRNA-30a-5p |
| | miR-195 : microRNA-195 |
| ATOPREG 1+2 | miR-155 : microRNA-155 |
| AUTIMREG 1+2+3 | miR-155 : microRNA-155 |
| | miR-181A : microRNA-181A |
| | miR-326 : microRNA-326 |
| BETACELL-REG 1+2+3 | miR-124a : microRNA-124a |
| | miR-375 : microRNA-375 |
| CELLCYCLING-REG 1+2+3 | miR-let-7a-d : microRNA-let-7a-d |
| | miR-let-7i : microRNA-let-7i |
| | miR-15b : microRNA-15b |
| | miR-34 : microRNA-34 |
| | miR-106b-25 : microRNA-106b-25 |
| | miR-335 : microRNA-335 |
| CHOLREG 1+2 | miR-122 : microRNA-122 |
| COLONMIR 1+2+3 | miR-9 : microRNA-9 |
| | miR-15b : microRNA-15b |
| | miR-17-3p : microRNA-17-3p |
| | miR-17-5p : microRNA-17-5p |
| | miR-18a : microRNA-18a |
| | miR-19 : microRNA-19 |
| | miR-20a : microRNA-20a |
| | miR-21 : microRNA-21 |
| | miR-30-3p : microRNA-30-3p |
| | miR-31 : microRNA-31 |
| | miR-92 : microRNA-92 |
| | miR-96 : microRNA-96 |
| | miR-101 : microRNA-101 |
| | miR-122 : microRNA-122 |
| | miR-124a : microRNA-124a |
| | miR-126 : microRNA-126 |
| | miR-129 : microRNA-129 |
| | miR-133b : microRNA-133b |
| | miR-135b : microRNA-135b |
| | miR-137 : microRNA-137 |
| | miR-143 : microRNA-143 |
| | miR-145 : microRNA-145 |
| | miR-181b : microRNA-181b |
| | miR-183 : microRNA-183 |
| | miR-191 : microRNA-191 |
| | miR-200c : microRNA-200c |
| | miR-328 : microRNA-328 |
| | miR-451 : microRNA-451 |
| CMV-REG 1+2 | miR-ULl12 : microRNA-UL112 |
| EBV-REG 1+2 | miR-BART2 : microRNA-BART2 |
| | miR-BART22 : microRNA-BART22 |
| | miR-cluster 1 BART : microRNA-cluster 1 BART |
| | miR-BHRF1 : microRNA-BHRF1 |
| | miR-155 : microRNA-155 |
| EMT-REG 1+2+3 | miR-23b : microRNA-23b |
| | miR-31 : microRNA-31 |
| | miR-141 : microRNA-141 |
| | miR-193b : microRNA-193b |
| | miR-155 : microRNA-155 |
| | miR-200 family (-200a,-200b,-200c, 429) : microRNA- |
| | 200 family |
| | miR-205 : microRNA-205 |
| | miR-222 : microRNA-222 |
| GLIOREG 1+2 | miR-34a : microRNA-34a |
| | miR-124 : microRNA-124 |
| | miR-137 : microRNA-137 |
| GLUCOREG 1+2 | miR-9 : microRNA-9 |
| | miR-29a : microRNA-29a |
| | miR-30d : microRNA-30d |
| | miR-34a : microRNA-34a |
| | miR-124a : microRNA-124a |
| | miR-146a : microRNA-146a |
| | miR-375 : microRNA-375 |
| HCV-REG 1+2 | miR-122 : microRNA-122 |
| ICA 1+2 | miR-1 : microRNA-1 |
| | miR-100 : microRNA-100 |
| | miR-133 : microRNA-133 |
| | miR-18B : microRNA-18B |
| | miR-195 : microRNA-195 |
| | miR-208 : microRNA-208 |
| | miR-21 : microRNA-21 |
| IgM-REG 1+2 | miR-9 : microRNA-9 |
| | miR-155 : microRNA-155 |
| | miR-184 : microRNA-184 |
| | miR-206 : microRNA-206 |
| | miR-363 : microRNA-363 |
| | miR-494 : microRNA-494 |
| | miR-542-3p : microRNA-542-3p |
| IRONREG | miR-210 : microRNA-210 |
| KERATOREG | miR-125b : microRNA-125b |
| | miR-21 : microRNA-21 |
| | miR-203 : microRNA-203 |
| | miR-146a : microRNA-146a |
| LYMREG Cl+2+3 | miR-15a : microRNA-15a |
| | miR-16-1 : microRNA-16-1 |
| | miR-29 : microRNA-29 |
| | miR-181 : microRNA-181 |
| | miR-155 : microRNA-155 |
| MAMMAREG 1+2+3 | MiR-10b : microRNA-10b |
| | miR-21 : microRNA-21 |
| | miR-27a : microRNA-27a |
| | miR-7 : microRNA-7 |
| | miR-17-5p : microRNA-17-5p |
| | miR-27b : microRNA-27b |
| | miR-101 : microRNA-101 |
| | miR-125a&b : microRNA-125a&b |
| | miR-126 : microRNA-126 |
| | miR-146 : microRNA-146 |
| | miR-205 : microRNA-205 |
| | miR-206 : microRNA-206 |
| | miR-335 : microRNA-335 |
| METABOLREG 1+2+3 | miR-9 : microRNA-9 |
| | miR-143 : microRNA-143 |
| METAREG 1+2+3 | miR-21 : microRNA-21 |
| | miR-335 : microRNA-335 |
| | miR-373 : microRNA-373 |
| | miR-520c : microRNA-520c |
| PLASMOREG 1+2+3 | miR-21 : microRNA-21 |
| | miR-30b : microRNA-30b |
| | miR-106b-25 cluster : microRNA-106b-25 cluster |
| | miR-181a&b : microRNA-181a&b |
| | miR-19a&b : microRNA-19a&b |
| | miR-17-92 cluster : microRNA-17-92 cluster |
| PROAPOPREG 1+2+3 | miR-195 : microRNA-195 |
| | miR-214 : microRNA-214 |
| PROSTAMIR 1+2 | miR-21 : microRNA-21 |
| | miR-125b : microRNA-125b |
| | miR-220/221 : microRNA-220/221 |
| | miR-34 family : microRNA-34 family |
| | miR-101 : microRNA-101 |
| | miR-126 : microRNA-126 |
| | miR-141 : microRNA-141 |
| | miR-146a : microRNA-146a |
| | miR-200c&b : microRNA-200c&b |
| | miR-330 : microRNA-330 |
| REDOXREG 1+2 | miR-29a,b,c : microRNA-29a,b,c |
| | miR-210 : microRNA-210 |
| REGOSTEOLYS 1+2 | miR-31 : microRNA-31 |
| | miR-126 : microRNA-126 |
| | miR-335 : microRNA-335 |
| | miR-10b : microRNA-10b |
| RYTHMOREG 1+2 | miR-1 : microRNA-1 |
| | miR-133 : microRNA-133 |
| STEMREG 1+2+3 | miR-315 : microRNA-315 |
| | miR-145 : microRNA-145 |
| | miR-let-7 : microRNA-let-7 |
| | miR-290 : microRNA-290 |
| | miR-295 : microRNA-295 |
| | miR-302 : microRNA-302 |
| | miR-LIN28 : microRNA-LIN28 |
| TH2-REG 1+2 | miR-126 : microRNA-126 |
| | miR-21 : microRNA-21 |
| WOUNDMIR | miR-105 : microRNA-105 |
| 1+2+3 | miR-125b : microRNA-125b |
| | miR-140 : microRNA-140 |
| | miR-146a : microRNA-146a |

Une composition selon la présente invention peut être obtenue par mise en oeuvre du procédé suivant à partir d'une solution mère contenant une nanomole d'ARNi dans 10 ml de solvant. Cette solution mère subira une méthodologie classique de dilution-succussion jusqu'à obtention de la composition homéopathique désirée. L'invention décrit donc un procédé de préparation d'une solution de type homéopathique caractérisé en ce qu'il comprend les étapes suivantes :
a) mettre en solution un ARN interférent dans un solvant pharmaceutiquement acceptable à raison de une nanomole pour 10 ml de solvant ;
b) faire subir à la solution ainsi obtenue une étape de succussion ;
c) diluer dans un solvant pharmaceutiquement acceptable la solution ainsi obtenue pour obtenir une dilution d'un facteur 10⁻¹ à 10⁻³ ;
d) répéter l'étape c) jusqu'à obtention d'une solution dynamisée diluée d'un facteur 10⁻⁸ par rapport à la concentration de départ.

On obtient une solution dynamisée qui sera utilisée soit sous forme liquide, soit sur un support solide, soit sur un support semi-solide. Dans le cas d'un support solide, la solution diluée et dynamisée est utilisée pour imprégner des granules, des globules de saccharose qui sont plus petits que les granules, ou de la poudre en flacon ou en sachet dose. Les compositions pharmaceutiques selon l'invention pourront être préparées par mise en oeuvre de la méthode décrite dans la demande de brevet FR84199948 (Laboratoires Boiron®). Dans le cas d'une utilisation sous forme liquide, la solution diluée et dynamisée est utilisée en gouttes ou ampoules buvables. Dans le cas d'un support semi-solide, la solution diluée et dynamisée est utilisée sous forme de suppositoires, ovules, capsules, liniments, onguents et pommades.

Parmi les solvants pharmaceutiquement acceptables on citera notamment l'alcool et l'eau.

Les formes solides sont en général administrées par voie sublinguale et sont à laisser fondre sous la langue pour une meilleure absorption.

Dans un mode de réalisation particulier, le procédé de préparation décrit ci-dessus met en oeuvre durant l'étape a) une ARNi sélectionné parmi les microARN (ARNmi), les petits ARN interférents (ARNsi) et les ARN en épingle à cheveu (ARNsh). En effet, on connaît les voies de synthèse des ces ARNi et il est donc aisé de les sélectionner pour cibler l'action que l'on souhaite avoir sur l'expression des gènes cibles.

Dans un mode de réalisation préféré, le procédé de préparation selon l'invention met en oeuvre durant l'étape a) un ARN interférent dont la taille est comprise entre 20 et 22 nucléotides. En effet, comme on l'a vu ci-dessus, les ARNmi et les ARNsi ont des tailles comprises entre 20 et 22 nucléotides suite à l'action des ribonucléases de type III appelées Dicer. Ces ARNi sont très spécifiques, reconnus et validés par la communauté scientifique, et leur fonction potentielle l'est également. Il n'y a donc aucune place pour le hasard dans le choix de ces ARNi.

Dans un second mode de réalisation préféré, le procédé de préparation selon l'invention met en oeuvre durant l'étape a) un ou plusieurs microARN (ARNmi).

Dans un mode de réalisation particulier, le procédé de préparation d'une solution de type homéopathique selon l'invention est caractérisé en ce qu'il comprend les étapes suivantes :
a) mettre en solution plusieurs ARN interférents dans un solvant pharmaceutiquement acceptable à raison de une nanomole pour 10 ml de solvant ;
b) faire subir à la solution ainsi obtenue une étape de succussion ;
c) diluer dans un solvant pharmaceutiquement acceptable la solution ainsi obtenue pour obtenir une dilution d'un facteur 10⁻¹ à 10⁻³ ;
d) répéter l'étape c) jusqu'à obtention d'une solution dynamisée diluée d'un facteur 10⁻⁸ par rapport à la concentration de départ.

La présente invention décrit enfin l'utilisation d'un ou plusieurs ARNi pour la fabrication d'une composition pharmaceutique selon la méthode homéopathique. Là encore, dans un mode de réalisation particulier, le ou les ARNi ont une taille comprise entre 20 et 22 nucléotides. Les ARNi sont préférentiellement choisis parmi les petits ARN interférents (ARNsi), les microARN (ARNmi) et les ARN en épingle à cheveu (ARNsh).

Les compositions pharmaceutiques selon l'invention sont particulièrement adaptées pour le traitement de pathologies caractérisées par une surexpression de protéines ou l'apparition de mutations aberrantes telles le cancer, les maladies neurodégénératives, certaines pathologies oculaires et certaines infections virales, etc..

### Exemples

Les différentes compositions pharmaceutiques de type homéopathique décrites ci-après sont préparées selon les principes classiques de l'homéopathie : préparation d'une solution fortement diluée obtenue par mise en oeuvre de manipulations classiques de type hahnémanien ou de type korsakovien, puis succussion (G. NETIEN et al., Galenica Médicaments Homéopathiques, édition 2, 1986) ou dynamisation. Les compositions détaillées ci-après sont élaborées suite à une analyse détaillée de l'état de santé du patient. Des analyses biologiques sont effectuées et analysées avant d'adapter la composition au patient (âge, taille, poids) mais également en fonction de son histoire médicale (pathologies passées, traitement(s) en cours, etc.) et de son état de santé au moment du traitement à l'aide d'une composition selon l'invention.

### Exemple 1 : compositions selon l'invention

### 1. Composition ayant un effet régulateur de troubles de la circulation coronarienne

Les deux compositions détaillées ci-après ont un effet sur des patients présentant des troubles de la circulation coronarienne. L'objectif de la composition pharmaceutique selon l'invention est de réguler ces troubles en agissant sur le matériel génétique qui est responsable de la cascade d'événements qui aboutit à la manifestation de ces troubles de la circulation coronarienne. Seules les dilutions des ARNmi sont indiquées.

| Code | COMPOSITION | DILUTIONS |
|---|---|---|
| Comp1 | VCAM-1 | / |
| | Hsp 60 | / |
| | IFN-γ | / |
| | IL-6 | / |
| | IL-12 | / |
| | IL-18 | / |
| | ROCK 1/2 | / |
| Comp2 | NO | / |
| | NF-κB | / |
| | TGF-ß | / |
| | IL-10 | / |
| | miR 135a | 17 CH |
| | miR 147 | 5 CH |

Légende pour la composition des différentes préparations ci-dessus:
Hsp 60 : pour « Heat Shock Protein 60 » ou protéine de stress sensible au choc thermique 60.
IFN-γ : pour interféron γ
IL-6 : pour « InterLeukin-6 » ou cytokine lymphocytaire de type 6.
IL-10 : pour « InterLeukin-10 » ou cytokine lymphocytaire de type 10.
IL-12 : pour « InterLeukin-12 » ou cytokine lymphocytaire de type 12.
IL-18 : pour « InterLeukin-18 » ou cytokine lymphocytaire de type 18.
miR 135a : pour microARN 135a.
miR 147 : pour microARN 147.
NF-κB : pour « nuclear factor kappa B ».
NO : pour « Nitric Oxide » ou monoxyde d'azote.
ROCK 1/2 : pour « RhO-associated Coiled-coil-containing protein Kinase 1&2 ».
TGF-β : pour « Tumor Growth factor-beta ».
VCAM-1 : pour « Vascular Cell Adhesion Molecule-1 » ou molécule 1 d'adhérence vasculaire cellulaire.

Les compositions 1 et 2 sont dosées en fonction des critères énoncés ci-dessus. Leur administration tend à réguler la circulation coronarienne et ainsi à prévenir toute pathologie qui pourrait survenir suite à des troubles de la circulation coronarienne tels des nécroses cellulaires pouvant aboutir à un infarctus.

### 2. Composition ayant un effet régulateur de proliférations lymphocytaires chroniques

Les trois compositions détaillées ci-après présentent un effet sur des patients ayant des troubles chroniques de la prolifération lymphocytaire. Seules les dilutions des ARNmi sont indiquées.

| Code | COMPOSITION | DILUTIONS |
|---|---|---|
| Comp3 | Bcl-2 | / |
| | Mcl-1 | / |
| | Survivin | / |
| | TRAF1 | / |
| | PI3-K | / |
| | IL-4 | / |
| | BLyS | / |
| | BAFF | / |
| | APRIL | / |
| | DNA *methyltransferase* | / |
| Comp4 | miR-15a | 5 CH |
| | miR-16-1 | 5 CH |
| | miR-29 | 5 CH |
| | miR-181 | 5 CH |
| | miR-155 | 17 CH |
| | CpG ODN | / |
| | IL-21 | / |
| | Bax | / |
| | NOXA | / |
| Comp5 | P73 gene | / |
| | Bcl-XL gene | / |
| | ZAP70 gene | / |
| | CD154 gene | / |
| | TCL1 gene | / |
| | Lyn | / |
| | NF^{κ} B | / |
| | bFGF | / |
| | VEGF | / |
| | P27^{KIP-1} | / |

Légende pour la composition des différentes préparations ci-dessus:
APRIL : pour « A Proliferation-Inducing Ligand »
BAFF : pour « B cell Activating Factor of the TNF Family »
Baux : pour « Bcl-2 Associated X protein »
Bcl-2 : pour « B cell lymphoma-2 »
Bcl-XL gène : pour « B cell lymphoma XL gene »
bFGF : pour « basic Fibroblast Growth Factor »
BLyS : pour « B lymphocyte Stimulator »
CD154 gène : pour gène codant pour CD154
CpG ODN : pour « unmethylated CG oligodeoynucleotides »
IL-4 : pour « InterLeukin-4 » ou cytokine lymphocytaire de type 4.
IL-21 : pour « InterLeukin-21 » ou cytokine lymphocytaire de type 21.
Lyn : pour « Lgk/Yes-related novel tyrosine kinase »
Mcl-1 : pour « Myeloid Cell Leukemia-1 »
NFκB : pour « Nuclear Factor kappa B »
miR-15a : pour microARN 15a
miR-16-1 : pour microARN 16-1
miR-29 : pour microARN 29
miR-181 : pour microARN 181
miR-155 : pour microARN 155
NOXA : pour désigner une protéine pré-apoptotique de la famille des protéines Bcl-2.
P27^{Kip-1} : pour « p27 ^{Kinase inhibitor protein-1} »
P73 gène : pour le gène codant pour la protéine 73.
PI3-K : pour « Phosphatidyl Inositol 3-Kinase »
Survivin : ou BIRC5 pour « Baculoviral Inhibitor of apoptosis Repeat-containing 5 ».
TCL1 gène : pour « T Cell Leukemia/Lymphoma gene »
TRAF1 : pour « Tumor necrosis factor Rzeceptor-Associated Factor-1 »
VEGF : pour « Vascular Endothelial Growth Factor »
ZAP70 gène : pour « Zeta-Associated Protein of 70 kDa gene »

L'indication clinique de la combinaison des compositions 3, 4 et 5 est l'apparition de troubles chroniques de la prolifération lymphocytaire.

### 3. Composition ayant un effet régulateur de la kératinisation cutanée

Les compositions détaillées ci-après présentent un effet sur des patients ayant des troubles de la kératinisation cutanée tel le psoriasis. Seules les dilutions des ARNmi sont indiquées.

| Code | COMPOSITION | DILUTIONS |
|---|---|---|
| Comp6 | VEGF | / |
| | TNF-α | / |
| | IFN-γ | / |
| | IL-6 | / |
| | IL-8 | / |
| | IL-17A | / |
| | STAT3 | / |
| | STAT4 | / |
| Comp7 | SOCS-3 | / |
| | IL-4 | / |
| | IL-5 | / |
| | IL-10 | / |
| | GATA 3 | / |
| | Foxp3 | / |
| | LL37 | / |
| | TLR 7 | / |
| | TLR 8 | / |
| | TLR 9 | / |
| Comp8 | HLA Cw06 gene | / |
| | CCHCR1 WWCC gene | / |
| | CDSN gene | / |
| | IL-12B (p40) gene | / |
| | IL-23R gene | / |
| | ZNF313 gene | / |
| | miR-203 | 30 CH |
| | miR-146a | 30 CH |

Légende pour la composition des différentes préparations ci-dessus:
CCHCR1 WWCC gène : pour « Coiled-Coil alpha HeliCal Rod protein 1 associated variant WWCC ».
CDSN gene : pour « Corneodesmosin gene allèle 5 ».
Foxp3 : pour « Forkhead boxp3 ».
GATA3 : pour facteur de transcription 3 se liant à la séquence GATA.
HLA Cw06 gene : pour « Human Leucocyte Antigen Cw06 gene ». IFN-γ : pour interféron γ
IL-4 : pour « InterLeukin-4 » ou cytokine lymphocytaire de type 4.
IL-5 : pour « InterLeukin-5 » ou cytokine lymphocytaire de type 5.
IL-6 : pour « InterLeukin-6 » ou cytokine lymphocytaire de type 6.
IL-8 : pour « InterLeukin-8 » ou cytokine lymphocytaire de type 8.
IL-10 : pour « InterLeukin-10 » ou cytokine lymphocytaire de type 10.
IL-12B (p40) gene : pour gène de l'interleukine 12B.
IL-17A : pour « InterLeukin-17A » ou cytokine lymphocytaire de type 17A.
IL-23R gene : pour gène du récepteur de l'interleukine 23. LL37 : pour « Cationic antimicrobial peptide ».
miR-203 : pour microARN 203.
miR-146a : microARN 146a.
SOCS-3 : pour « Suppressor Of Cytokine Signaling-3 ».
STAT3 : pour « Signal Transducer and Activator of Transcription 3 ».
STAT4 : pour « Signal Transducer and Activator of Transcription 4 ».
TLR 7 : pour « Toll Like Receptor 7 ».
TLR 9 : pour « Toll Like Receptor 9 ».
TNF-α : pour « Tumor Necrosis Factor-alpha »
VEGF : pour « Vascular Endothelial Growth Factor » ou facteur de croissance de l'endothélium vasculaire.
ZNF313 gène : pour gène codant pour « Zinc-finger protein 313 » la protéine en doigt de zinc 313.

L'indication clinique de la combinaison des compositions 6, 7 et 8 est le psoriasis ainsi que toutes les hyperkératoses et kératodermies palmoplantaires.

### 4. Composition ayant un effet régulateur du taux de plasmocytes

Les compositions détaillées ci-après présentent un effet sur des patients ayant des troubles du taux de plasmocytes. Seules les dilutions des ARNmi sont indiquées.

| Code | COMPOSITION | DILUTIONS |
|---|---|---|
| Comp9 | IL-6 | / |
| | STAT3 | / |
| | Hsp90 | / |
| | SOCS-1 | / |
| | BIM | / |
| | PTEN gene | / |
| | P21Waf1/Cipl gene | / |
| | MiR-15a | 5 CH |
| | miR-16 | 5 CH |
| | PCAF gene | / |
| Comp10 | EIF2C2/AGO2 | / |
| | miR-21 | 30 CH |
| | miR-30b | 30 CH |
| | miR-106b-25 cluster | 30 CH |
| | miR-32 | 30 CH |
| | miR-181a&b | 30 CH |
| | miR-19a&b | 30 CH |
| | miR-17-92 cluster | 30 CH |
| | PI3k/Akt/mTOR | / |
| | IGF-1 | / |

Légende pour la composition des différentes préparations ci-dessus:
BIM : pour « B-cell lymphoma 2 Interacting Mediator of cell death ».
EIF2C2/AG02 gène : pour gene de "eukaryotic translation initiation factor 2C, 2/ Argonaute 2".
Hsp 90 : pour « Heat Shock Protein 90 » ou protéine de stress sensible au choc thermique 90.
IGF-1 : pour « Insulin like Growth Factor 1 ».
IL-6 : pour « InterLeukin-6 » ou cytokine lymphocytaire de type 6.
miR-15a : pour miroARN 15a.
miR-16 : pour microARN 16.
miR-17-92 cluster : pour « microARN 17-92 cluster ». miR-19a&b : pour microARN 19a&b.
miR-21 : pour microARN 21.
miR-30b : pour microARN 30b.
miR-32 : pour microARN 32.
miR-106b-25 cluster : pour « microARN 106b-25 cluster ». miR-181a&b : pour microARN 181a&b.
PCAF gene : pour « p300-CBP-Associated Factor ». PI3k/Akt/mTOR : pour « PhosphatidylInositol 3-Kinase/Akt/mammalian Target Of Rapamycin ».
PTEN gene : pour « Phosphatase and TENsin homolog gene ». SOCS-1 : pour « Suppressor Of Cytokine Signaling-1 ».
STAT3 : pour « Signal Transducer and Activator of Transcription 3 ».

Les compositions 9 et 10 sont particulièrement appropriées pour la régulation du taux de plasmocytes. Les indications cliniques sont les gammopathies monoclonales bénignes, les myélomes multiples ainsi que les plasmocytomes.

### Exemple 2 : cas cliniques

### 1. Régulation du taux de thymidine kinase

Sujet masculin de 45 ans, chef d'entreprise venant consulter pour établir un bilan immunitaire élargi car il se sent particulièrement fatigué.

Le bilan immunitaire réalisé par des laboratoires spécialisés montre des éléments en faveur d'un processus néoplasique encore latent. Cette hypothèse est confirmée par une augmentation significative du taux de thymidine kinase plasmatique.

Le traitement proposé à l'aide d'une composition pharmaceutique selon l'invention consiste en la combinaison de deux compositions visant à réguler le taux de thymidine kinase plasmatique.

Ces deux compositions BIMUREG® contiennent notamment les microARN suivants :

| BIMUREG® | microARN (ARNmi) entrant dans la composition |
|---|---|
| APOPMIR-PLUS | miR-let7/miR-98 : microRNA-let7/microRNA-98 |
| | miR-1 : microRNA-1 |
| | miR-29a,b,c : microRNA-29a,b,c |
| | miR-34a,b,c : microRNA-34a,b,c |
| | miR-15/16 : microRNA-15/16 |
| | miR-101 : microRNA-101 |
| | miR-215 : microRNA-215 |
| | miR-192 : microRNA-192 |
| | miR-127 : microRNA-127 |
| PROAPOPREG 1+2+3 | miR-195 : microRNA-195 |
| | miR-214 : microRNA-214 |

La durée de traitement est de quatre mois. Un nouveau bilan immunitaire est réalisé après cette période de traitement. D'une part le patient se sent beaucoup moins fatigué et d'autre part son taux de thymidine kinase est complètement normalisé pour un homme actif de 45 ans.

### 2. Régulation du développement cellulaire

Sujet féminin de 78 ans atteint d'un cancer du colon ayant métastasé dans le foie, le péritoine et les poumons malgré un traitement conventionnel. État de santé général déplorable, importante perte de poids.

Le protocole de chimiothérapie conventionnel est un échec selon le propre aveu de l'oncologue de ce sujet. Ce protocole est cependant maintenu. En sus, un traitement BIMUREG est prescrit.

| BIMUREG® | microARN (ARNmi) entrant dans la composition |
|---|---|
| APOPMIR-PLUS | miR-let7/miR-98 : microRNA-let7/microRNA-98 |
| | miR-1 : microRNA-1 |
| | miR-29a,b,c : microRNA-29a,b,c |
| | miR-34a,b,c : microRNA-34a,b,c |
| | miR-15/16 : microRNA-15/16 |
| | miR-101 : microRNA-101 |
| | miR-215 : microRNA-215 |
| | miR-192 : microRNA-192 |
| | miR-127 : microRNA-127 |
| AHT 1+2+3 | miR-155 : microRNA-155 |
| PROAPOPREG 1+2+3 | miR-195 : microRNA-195 |
| | miR-214 : microRNA-214 |

La durée de traitement est de trois ans. L'état général du sujet est grandement amélioré avec une prise de poids d'une dizaine de kilos. Les nodules métastasiques sont tous en voie de calcification.

### 3. Régulation de syndromes oedémateux

Sujet féminin de 89 ans atteint d'une insuffisance cardiaque ne pouvant plus être maitrisée par les traitements conventionnels. Développement d'un syndrome oedémateux et dyspnéique.

Le traitement BIMUREG envisagé est le suivant :

| BIMUREG® | microARN (ARNmi) entrant dans la composition |
|---|---|
| ICA 1+2 | miR-1 : microRNA-1 |
| | miR-100 : microRNA-100 |
| | miR-133 : microRNA-133 |
| | miR-18B : microRNA-18B |
| | miR-195 : microRNA-195 |
| | miR-208 : microRNA-208 |
| | miR-21 : microRNA-21 |

En l'espace de quelques semaines les oedèmes se résorbent jusqu'à disparaître. L'état de santé général du sujet s'améliore rapidement.

### 4. Régulation de l'évolution de la démyélinisation Sujet féminin de 40 ans atteint d'une sclérose en plaques.

L'évolution de la maladie est progressive et le sujet refuse les traitements proposés.

Le traitement BIMUREG proposé contient les microARN suivants :

| BIMUREG® | microARN (ARNmi) entrant dans la composition |
|---|---|
| AUTIMREG 1+2+3 | miR-155 : microRNA-155 |
| | miR-181A : microRNA-181A |
| | miR-326 : microRNA-326 |
| APOPMIR-MINUS | miR-17-92 : microRNA-17-92 |
| | miR-21 : microRNA-21 |
| | miR-106b-25 : microRNA-106b-25 |
| | miR-133 : microRNA-133 |
| | miR-143 : microRNA-143 |
| | miR-145 : microRNA-145 |
| | miR-155 : microRNA-155 |
| | miR-206 : microRNA-206 |
| | miR-210 : microRNA-210 |
| | miR-221/222 : microRNA-221/222 |
| | miR-330 : microRNA-330 |

En deux ans la sclérose en plaques voit sa progression ralentie voire bloquée depuis la prise de la composition BIMUREG ci-dessus. Les images réalisées par IRM confirment qu'aucune nouvelle zone de démyélinisation n'est apparue et que les lésions anciennes connaissent une bonne cicatrisation.

### 5. Régulation d'un état inflammatoire

Sujet féminin de 45 ans atteint d'une hépatite auto-immune, d'une thyroïdite de même nature et d'une cholangite sclérosante. Sujet soumis depuis 10 ans à une corticothérapie hautement dosée. Le profil protéique montre cependant toujours une inflammation chronique singulièrement active.

Le traitement BIMUREG proposé contient les microARN suivants :

| BIMUREG® | microARN (ARNmi) entrant dans la composition |
|---|---|
| AIF 1+2+3 | miR-146a : microRNA-146a |
| AUTIMREG 1+2+3 | miR-155 : microRNA-155 |
| | miR-181A : microRNA-181A |
| | miR-326 : microRNA-326 |

En moins de trois mois l'inflammation chronique est totalement neutralisée sur le plan biologique et clinique. Le sujet ressent une nette amélioration de son état de santé général.

## Revendications

1. Composition pharmaceutique préparée selon la méthodologie homéopathique **caractérisée en ce qu'**elle contient un ou plusieurs acides ribonucléiques interférents (ARNi).

2. Composition pharmaceutique selon la revendication 1, **caractérisée en ce qu'**elle contient un ou plusieurs acides ribonucléiques interférents (ARNi) double brin.

3. Composition pharmaceutique selon la revendication 1, **caractérisée en ce qu'**elle contient un ou plusieurs acides ribonucléiques interférents (ARNi) simple brin.

4. Composition pharmaceutique selon l'une quelconque des revendications 1 à 3, **caractérisée en ce qu'**elle contient un ou plusieurs acides ribonucléiques interférents (ARNi) dont la taille est comprise entre 20 et 22 nucléotides.

5. Composition pharmaceutique selon l'une quelconque des revendications 1 à 3, **caractérisée en ce que** le ou les acides ribonucléiques interférents sont choisis parmi les petits ARN interférents (ARNsi), les microARN (ARNmi) et les ARN en épingle à cheveu (ARNsh).

6. Composition pharmaceutique selon l'une quelconque des revendications 1 à 4, **caractérisée en ce que** le ou les acides ribonucléiques interférents sont choisis parmi les petits ARN interférents (ARNsi) et les microARN (ARNmi).

7. Composition pharmaceutique selon l'une quelconque des revendications précédentes, **caractérisée en ce que** le ou les acide (s) ribonucléique(s) interférent(s) est(sont) un(des) microARN(s) (ARNmi).

8. Composition pharmaceutique selon l'une quelconque des revendications précédentes, **caractérisée en ce que** le ou les acide(s) ribonucléique(s) interférent(s) est(sont) un(des) petit(s) ARN(s) interférent (ARNsi).

9. Procédé de préparation d'une solution de type homéopathique **caractérisé en ce qu'**il comprend les étapes suivantes :
a) mettre en solution un ARN interférent dans un solvant pharmaceutiquement acceptable à raison de une nanomole pour 10 ml de solvant ;
b) faire subir à la solution ainsi obtenue une étape de succussion ;
c) diluer dans un solvant pharmaceutiquement acceptable la solution ainsi obtenue pour obtenir une dilution d'un facteur 10⁻¹ à 10⁻³ ;
d) répéter l'étape c) jusqu'à obtention d'une solution dynamisée diluée d'un facteur 10⁻⁸ par rapport à la concentration de départ.

10. Procédé selon la revendication 9, **caractérisé en ce que** l'ARN interférent de l'étape a) est sélectionné parmi les microARNs, les petits ARN interférents et les ARN en épingle à cheveux.

11. Procédé selon la revendication 9, **caractérisé en ce que** l'ARN interférent de l'étape a) a une taille comprise entre 20 et 22 nucléotides.

12. Procédé selon l'une quelconque des revendications 9 à 11, **caractérisé en ce que** l'ARN interférent de l'étape a) est un microARN.

13. Procédé selon l'une quelconque des revendications 9 ou 11, **caractérisé en ce que** l'ARN interférent de l'étape a) est un petit ARN interférent.

14. Procédé de préparation d'une solution de type homéopathique **caractérisé en ce qu'**il comprend les étapes suivantes :
a) mettre en solution plusieurs ARN interférents dans un solvant pharmaceutiquement acceptable à raison de une nanomole pour 10 ml de solvant ;
b) faire subir à la solution ainsi obtenue une étape de succussion ;
c) diluer dans un solvant pharmaceutiquement acceptable la solution ainsi obtenue pour obtenir une dilution d'un facteur 10⁻¹ à 10⁻³ ;
d) répéter l'étape c) jusqu'à obtention d'une solution dynamisée diluée d'un facteur 10⁻⁸ par rapport à la concentration de départ.
